# EUROPEAN PATENT APPLICATION

(11) **EP 4 830 736 A1**
(43) Date of publication of application: **28.10.2026**
(21) Application number: 25305598.2
(22) Date of filing: 25.04.2025
(51) Int. Cl.: A61K 51/04, A61K 51/08, A61K 103/40, C07D 273/08, C07F 9/38, C07K 5/04

(54) **DIAZA-18-CROWN-6 DERIVATIVES USEFUL FOR CHELATING ACTINIUM, CONJUGATE AND ACTINIUM CHELATE COMPRISING THE SAME, AND USES THEREOF**

(71) Applicant: Orano, 92320 Chatillon (FR)
(72) Inventor: Mindt, Thomas, 1090 Vienna (AT); Gumiela, Magdalena, 1090 Vienna (AT); Southwell, James, 75005 Paris (FR); Gasser, Gilles, 75005 Paris (FR); Cowell, Joseph, 75005 Paris (FR)
(74) Representative: Gevers & Orès

(57) **Abstract**

The present invention relates to diaza-18-crown-6 derivatives which are able to form stable chelates with radioisotopes, such as actinium ions (Ac³⁺), in physiological media and which can be functionalized with a moiety for a covalent bonding reaction, and their use as radiopharmaceutical agents.

## Description

The present invention belongs to the field of chelation of radioisotopes in physiological media.

More specifically, it relates to diaza-18-crown-6 derivatives which are able to form stable chelates with actinium ions (Ac³⁺) in physiological media and which can be functionalized with a moiety for a covalent bonding reaction. This moiety allows, for example, covalently bonding the diaza-18-crown-6 derivative to a biological cell-targeting moiety in view of preparing a radiopharmaceutical. It also relates to a conjugate comprising the diaza-18-crown-6 derivative covalently bonded to a biological cell-targeting moiety and, notably, to a cancer cell-targeting moiety.

It further relates to a chelate comprising actinium and, notably, actinium-225, chelated by the conjugate.

It still further relates to uses of the diaza-18-crown-6 derivative, the conjugate and the chelate.

### Background of the Invention

The use of α-emitting radionuclides in targeted alpha therapy (TAT) has great potential for cancer treatment. Such systems are typically composed of three parts: the radionuclide, its chelator and a covalently attached targeting biomolecule (e.g. peptide or antibody). α particles travel short distances corresponding to only a few cell diameters in human tissue (<0.1 mm) in contrast to that of β particles (2-11 mm), in theory providing improved tissue selectivity. The energy deposited by an α-compared to a β-particle can be even 1000 times greater per unit distance travelled. The resulting high linear energy transfer (LET) leads to substantial DNA double-strand breaks causing cell death. Thus, TAT offers the selective irradiation of cancer cells with minimal impact on surrounding healthy tissue, which is not only beneficial for efficient tumor treatment but also a promising approach for treating micrometastases.

Unfortunately, the use of α-emitters in medicine is limited by low radionuclide availability, high cost, specific physicochemical properties of the respective nuclides (e.g. short half-life) and the lack of compatible chelator systems.

Diaza-18-crown-6 derivatives have been previously described in WO2021/250240 for chelating radium.

It remains the need to have improved chelators for TAT.

### Detailed description

Inventors have now identified specific diaza-18-crown-6 derivatives that are able to chelate Ac³⁺ ions efficiently, leading to complexes that are very stable in physiological media and have improved solubility. In addition, these advantageous properties are preserved even when such diaza-18-crown-6 derivatives are covalently bonded to a biological cell-targeting moiety such as a cancer cell-targeting moiety.

The present invention thus relates to a chelate comprising a radioisotope and specific diaza-18-crown-6 derivatives, wherein said diaza-18-crown-6 derivatives are compounds of general formula (I): 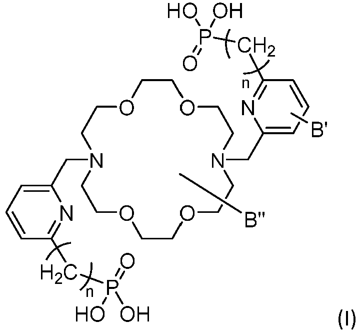 wherein n is independently chosen from 0, 1, 2, 3 or 4; preferably, n is 0 or 1; and wherein
B' is a hydrogen atom, B or -L-C, and
B" is a hydrogen atom or B,

B being preferably a saturated or unsaturated, acyclic hydrocarbon group comprising 1 to 12 carbon atoms with optionally one or more heteroatoms, and comprising a reactive group chosen from the carboxyl, acyl halide, acid anhydride, hydroxyl, aldehyde, azide, ketone, thiol, amine, amide, halogeno, ester, ether, vinyl, alkynyl (for example, propargyl), imide, maleimide and oxime groups; N-hydroxysuccinimide esters, squaric esters or isothiocyanate;
L being a linker; and
C being a biological cell-targeting moiety.
B' can be linked to any unsubstituted carbon atom of the pyridine ring and B" can be linked to any carbon atom of the diaza-18-crown-6.

According to one embodiment of the invention, compounds of general formula (I) are such that B' and B" are hydrogen atoms and said compounds have general formula (II):

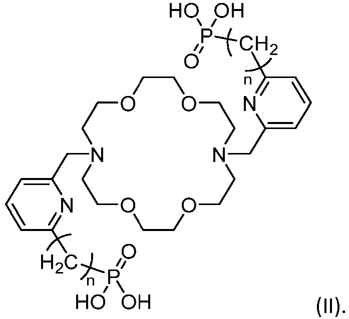

Preferably, n is 0 or 1; accordingly, the compounds of formula (II) are respectively MacroPhos of structure (IIA): 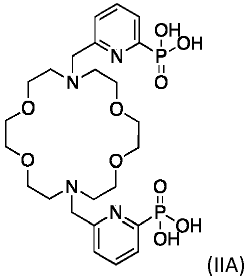 or MacroMePhos of structure (IIB):

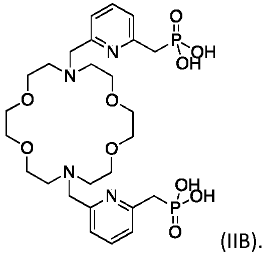

According to another embodiment of the invention, compounds of general formula (I) are such that when B' is B or -L-C, then B" is a hydrogen atom and when B' is a hydrogen atom, then B" is B. According to this another embodiment, compounds may have general formula (III): 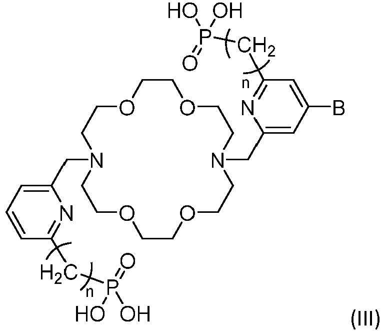 wherein n is independently chosen from 0, 1, 2, 3 or 4; preferably, n is 0 or 1; and
wherein B is preferably a saturated or unsaturated, acyclic hydrocarbon group comprising 1 to 12 carbon atoms with optionally one or more heteroatoms, and comprising a reactive group chosen from the carboxyl, acyl halide, acid anhydride, hydroxyl, aldehyde, azide, ketone, thiol, amine, amide, halogeno, ester, ether, vinyl, alkynyl (for example, propargyl), imide, maleimide and oxime groups; N-hydroxysuccinimide esters, squaric esters or isothiocyanate.

By "saturated or unsaturated, acyclic hydrocarbon group comprising 1 to 12 carbon atoms and optionally one or more heteroatoms", is meant any saturated, linear or branched, hydrocarbon group, i.e. any alkyl group, comprising 1, 2, 3, 4, 5, 6, 7, 8, 9, 10, 11 or 12 carbon atoms or any unsaturated, linear or branched, hydrocarbon group, i.e. any alkenyl or alkynyl group, comprising 2, 3, 4, 5, 6, 7, 8, 9, 10, 11 or 12 carbon atoms, the chain of which may be interrupted by one or more heteroatoms or be substituted by one or more heteroatoms.

By "heteroatom", is meant any atom other than carbon and hydrogen atoms, the heteroatom being typically a nitrogen, oxygen or sulphur atom.

The reactive group of the moiety B is preferably a terminal carboxyl group (-COOH) or amine group (-NH₂).

Hence, the moiety B may notably be a group of formula:
* -(CH₂)ₚ-NH₂, where p is an integer from 1 to 10 and, preferably, from 1 to 6,
* -(CH₂)_{q}-COOH, where q is an integer from 1 to 10 and, preferably, from 1 to 6,
* -(CH₂)ᵣ-O-(CH₂)ₛ-NH₂ , where r and s, identical or different, are integers from 1 to 5 and, preferably, equal to 1, 2 or 3,
* -(CH₂)ᵣ-O-(CH₂)ₛ-COOH, where r and s, identical or different, are integers from 1 to 5 and, preferably, equal to 1, 2 or 3,
* -(CH₂)ᵣ-NH-CO-(CH₂)ₛ-NH₂, where r and s, identical or different, are integers from 1 to 5 and, preferably, equal to 1, 2 or 3, or
* -(CH₂)ᵣ-NH-CO-(CH₂)ₛ-COOH, where r and s, identical or different, are integers from 1 to 5 and, preferably, equal to 1, 2 or 3.

Alternatively, but still according to this another embodiment, compounds of formula (I) have general formula (IV): 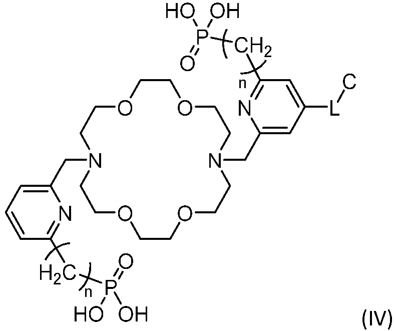 wherein n is independently chosen from 0, 1, 2, 3 or 4, preferably, n is 0 or 1; and wherein
L is a linker; and
C is a biological cell-targeting moiety.

By "biological cell-targeting moiety", is meant any moiety able to specifically recognize and bind to biological cells. Such a moiety may be a biomolecule (i.e. a molecule which is naturally present in living beings) or with a synthetic molecule which mimics structurally and/or functionally a biomolecule.

Thus, the targeting moiety for biological cells may notably comprise or be constituted by a monoclonal antibody, a monoclonal antibody fragment, a genetically engineered antibody, a protein, a peptide, a carbohydrate, an oligonucleotide such as an aptamer, a vitamin, a hormone, a neurotransmitter, a steroid, a nanoparticle, a vesicle such as a liposome, a growth factor, an agonist or antagonist ligand of a cell receptor such as a membrane receptor.

According to a particular embodiment of the invention, the compound of formula (II) may be directly bonded to the moiety C.

However, the compound of formula (II) may also be bonded to the moiety C via a spacer arm to which the moiety C is already bonded or will be later bonded, in which case L is the atom group formed by the rest of the moiety B together with the rest of the spacer arm. Hereto, by "rest of the spacer arm", is meant the atom group belonging to the spacer arm which remains after being bonded, on the one hand, to the compound of formula (II) and, on the other hand, to the moiety C. The spacer arm may be chosen from a large number of compounds, as long as they have at least two reactive groups capable of chemically reacting with, on the one hand, the reactive group of the compound of formula (II) and, on the other hand, a reactive group belonging to the moiety C, and do not adversely affect the solubility of the conjugate in aqueous media and, notably, in physiological media.

Thus, for example, the spacer arm may notably be a polyethylene glycol such as, for example, PEG₂, PEG₄, PEG₆ or PEG₁₂, an oligopeptide (preferably of no more than 10 amino acids), a polyamine, a polyol, an amino-alkanoic acid such as 4-aminobutanoic acid, 5-aminopentanoic acid or 6-aminohexanoic acid, an alkanedioic acid such as butanedioic acid, pentanedioic acid or hexanedioic acid, a group comprising at least one cycle (which may be a cycloalkyl such as a cyclopentyl or cyclohexyl, a saturated or unsaturated heterocycle such as a piperidinyl, pyridinyl, phenyl or triazolyl cycle) together with two reactive functions such as 4-amino-1-carboxymethylpiperidine or 4-(2aminoethyl)-1-carboxymethyl piperazine, and combinations thereof.

If the compound of formula (IV) is intended to be used, after chelation of actinium, as a radiopharmaceutical for treating a prostate cancer and, such as, a castration-resistant prostate cancer by targeted a-radiotherapy, then the targeting moiety for biological cells can be:
- either a bombesin receptor (GRPR) antagonist peptide such as, for example, one of those which are disclosed in WO2009/109332 and notably the peptide known as JMV594 (of formula: DPhe-Gln-Trp-Ala-Val-Gly-His-Sta-Leu-NH₂);
- or a ligand of the prostate specific membrane antigen (PSMA) such as, for example, one of those which are disclosed in WO2009/026177 and notably one of those which are urea of two amino acids.

An example of compound of formula (IV) is: 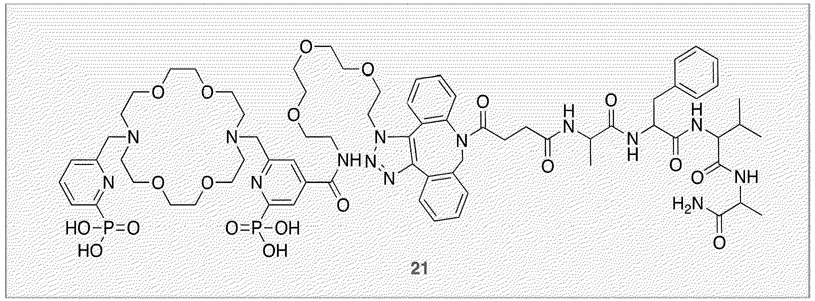 that comprises MacroPhos conjugated with the peptide AVFA (Alanine-Valine-PHEnylalanine-Alanine).

The radioisotope is selected in the group consisting of actinium such as actinium-225, terbium, such as terbium-155 or terbium-161, zirconium, such as zirconium-89, lutetium, such as lutetium-177, lanthanum, such as lanthanum-132 or lanthanum-135 and radium, such as radium-223 or radium-224; preferably, actinium-225.

In a particular embodiment, the present invention relates to a chelate comprising a radioisotope selected in the group consisting of actinium such as actinium-225, terbium, such as terbium-155 or terbium-161, zirconium, such as zirconium-89, lutetium, such as lutetium-177, lanthanum, such as lanthanum-132 or lanthanum-135 and radium, such as radium-223 or radium-224; preferably, actinium-225; and a compound of formula (I), including compounds of formulae (II), (III) and (IV), for use for the treatment of cancer.

According to this embodiment, such chelate may be used as a radiopharmaceutical and, notably, for treating cancer by targeted α-radiotherapy, which cancer can be melanoma, glioblastoma, breast, pancreas, lung, gastro-intestinal, gynecological or prostate cancer such as a castration-resistant prostate cancer.

According a specific embodiment, the present invention relates to compounds of general formula (II):

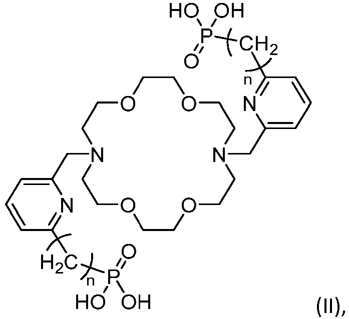

In particular to MacroMePhos of structure (IIB): 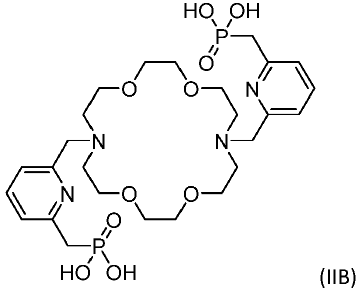 and their use to prepare compounds of formulae (III) and (IV).

The general synthesis route of compounds of formula (I) of the invention is as described in WO2021/250240.

The radiolabelling of any of the compounds (I), (II) or (III) of the invention with a radioisotope, in particular with actinium, may be performed as follows: said compound, optionally in presence of gentisic acid or ascorbic acid, and in presence of ²²⁵Ac³⁺ are added to ammonium acetate buffer (pH 6).

### Figures

Figure 1: TLC chromatogram of [²²⁵Ac][Ac(MacroPhos)], RCY=100 %.
Figure 2: Drawn SEC chromatograms of [²²⁵Ac][Ac(MacroPhos)] challenged with human serum (1:100 vol/vol).
Figure 3: TLC chromatogram of [²²⁵Ac][Ac(MacroPhos-AVFA)], RCY=99% after 5 min.
Figure 4: Drawn SEC chromatograms of [²²⁵Ac][Ac(MacroPhos-AVFA)] challenged with human serum (1:100 vol/vol).

### Examples

### Instrumentation:

### NMR

¹H, ¹³C(¹H), ³¹P(¹H), DEPT 135, HSQC, COSY, HMBC and NOESY NMR spectra are recorded on at room temperature (303 K) either on a Bruker Avance III HD 400 MHz spectrometer (equipped with a BBFO Plus Smartprobe, ¹H: 400 MHz ; ¹³C: 100 MHz ; ³¹P: 162 MHz), or on a Bruker Avance Neo 500 MHz spectrometer (equipped with a BBFO Smartprobe BB & ¹⁹F/¹H, 1H: 500 MHz ; ¹³C: 125 MHz ; ³¹P: 202 MHz). ³¹P(¹H) NMR chemical shifts are reported in ppm and externally referenced to 85% H₃PO₄ at 0 ppm. Ile de France Region is gratefully acknowledged for financial support of 500 MHz NMR spectrometer of Chimie ParisTech in the framework of the SESAME equipment project (n°16016326).

Multiplicity abbreviations are as follows; s for singlet, d for doublet, t for triplet, q for quartet qn for quintet, sx for sextet, sp for septet, dd for double doublet, td triple doublet, ddd for doublet of doublet of doublets, m for multiplet, br for broad and app for apparent. NMR assignments were aided by various combinations of DEPT 135, HSQC, COSY, HMBC and NOESY experiments, where required.

### HRMS

ESI-MS experiments are carried out using an LTQ-Orbitrap XL (Thermo Fisher Scientific, Courtaboeuf, France) operated in positive or negative ionization mode, with a spray voltage at 3.6 kV and an ion transfer capillary temperature of 275°C. Sheath gas flow rate was set at 30. Applied voltages depended on the theoretical mass of the desired product within the sample (see Table 1) for the ion transfer capillary and the tube lens, respectively. Detection was achieved in the Orbitrap with a resolution of 100,000 (at m/z 400) where the m/z range in profile mode depended (see Table 1) on the applied voltage. The spectra were recorded using the acquisition software XCalibur 2.1 (Thermo Fisher Scientific, Courtaboeuf, France). Typical automatic gain control (AGC) and maximum injection time were 2.10⁵ ions and 500 ms. 1 µscan was acquired. Internal lockmass was enabled using in-house reference compounds. 1-10 µL (10 µg.mL⁻¹) were injected using a Thermo Finnigan Surveyor HPLC system (Thermo Fisher Scientific, Courtaboeuf, France) with a continuous infusion of methanol at 60 µL.min⁻¹.

APCI-MS experiments were carried out using an LTQ-Orbitrap XL (Thermo Fisher Scientific, Courtaboeuf, France) and operated in positive or negative ionization mode, with a vaporizer temperature at 400°C and a corona discharge at 5 µA. Values were set at 45, 5 and 5 for sheath, auxiliary and sweep gas flow, respectively. Applied voltages depended on the theoretical mass of the desired product within the sample (see Table 1) for the ion transfer capillary and the tube lens, respectively. The ion transfer capillary was held at 275°C. Detection was achieved in the Orbitrap with a resolution of 100,000 (at m/z 400) where the m/z range in profile mode depended (see Table 1) on the applied voltage. The spectra were recorded using the acquisition software XCalibur 2.1 (Thermo Fisher Scientific, Courtaboeuf, France). Typical automatic gain control (AGC) and maximum injection time were 2.10⁵ ions and 500 ms. 1 µscan was acquired. Internal lockmass was enabled using in-house reference compounds. 1-10 µL (10 µg.mL⁻¹) were injected using a Thermo Finnigan Surveyor HPLC system (Thermo Fisher Scientific, Courtaboeuf, France) with a continuous infusion of methanol at 60 µL.min⁻¹.

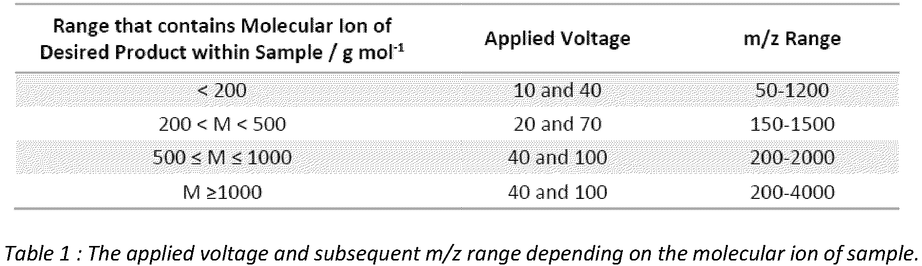

### I- Synthesis of chelator compounds of formula (I) of the invention

The synthesis of MacroPhos (also named compound 6) is achieved following the same synthetic route outlined by Fiszbein *et al.* in 2021 (DOI: 10.1021/acs.inorgchem.1c01269).

The synthesis of the novel compound MacroMePhos (also named compound 7) is outlined below:, 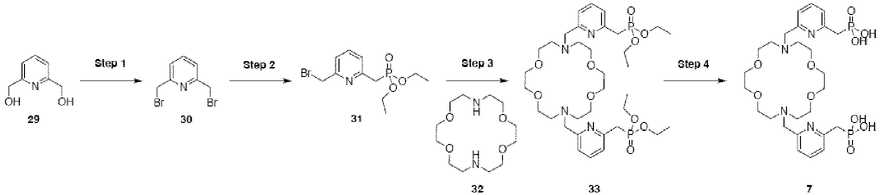 and is detailed below:

### Step 1:

This synthesis is based on conditions reported by Abda *et al.* in 2014 (DOI: 10.1039/C4OB01514B). To a stirred solution of **29** (1.05 g, 7.57 mmol) in dry DMF (100 mL) at 0°C is added phosphorous tribromide (1.64 mL, 17.40 mmol) dropwise. The reaction mixture is then allowed to warm to room temperature and left stirring for 18h under these conditions. After this time, the reaction is quenched with the minimum water, and the solvent is removed *in vacuo.* The resulting residue is dissolved in water (50 mL), and then extracted using diethyl ether (50 mL, x4). The solvent from the combined organic layers is then removed *in vacuo,* to give the desired compound, **30,** as a white solid (1.60 g, 80% yield).

**Rf:** 0.79 in EtOAc.

**HRMS (ESI+):** [M+H]+ Calcd. for C7H7Br2N 263.9018. Found 263.902; (Error: 0.8 ppm).

**¹H NMR:** (400 MHz, CDCl3) δ: 7.70 (t, *J* = 7.5 Hz, 1H), 7.37 (d, *J* = 7.5 Hz, 2H), 4.53 (s, 4H).

### Step 2:

A stirred solution of **30** (1.07 g, 4.04 mmol) in dry toluene (50 mL) at room temperature with triethylphosphite (738 mg, 4.40 mmol) and zinc bromide (182 mg, 808 µmol) is heated at reflux for 24 h. The resulting reaction mixture is then adsorbed onto celite and purified by automated flash chromatography by elution with EtOAc, to give the desired compound, **31,** as a clear, colourless oil (453 mg, 44% yield).

**Rf:** 0.66 in dichloromethane:MeOH (9:1).

**HRMS (ESI+):** not stable enough for submission.

**¹H NMR:** (400 MHz, CDCl3) δ: 7.72 (t, *J* = 7.5 Hz, 1H), 7.42 - 7.38 (dd, *J* = 7.5, 2.0 Hz, 1H), 7.35 (dd, *J* = 7.5, 2.0 Hz, 1H), 4.65 (s, 2H), 4.26 - 4.04 (m, 4H), 3.56 (d, *J =* 22.0 Hz, 2H), 1.31 (t, *J =* 7.0 Hz, 6H). **³¹P NMR:** (162 MHz, CDCl3) δ: 25.6 (s).

**¹³C NMR:** (101 MHz, CDCl3) δ: 157.2, 152.5, 138.4, 124.4, 122.5, 63.4, 36.1 (d, *J =* 135.0 Hz), 33.7, 16.4.

### Step 3:

A stirred solution of **31** (115 mg, 358 µmol), **32** (47 mg, 179 µmol), and potassium carbonate (49 mg, 358 µmol) in dry acetonitrile (30 mL) is heated at reflux for 18 h. After this time, the reaction solvent is removed *in vacuo,* and redissolved in dichloromethane (30 mL). This solution is washed with saturated NaHCO₃ (aq) (20 mL, x3), to give the desired compound, **33,** as a clear, colourless oil (107 mg, 80% yield).

**HRMS (ESI+):** [M+H]+ Calcd. for C34H58N4O10P2 745.3701. Found 745.3712; (Error: 1.9 ppm).

**¹H NMR:** (400 MHz, CDCl3) δ: 7.58 (t, *J* = 7.5 Hz, 2H), 7.43 (d, *J* = 8.0 Hz, 2H), 7.23 (dd, J = 7.5, 2.0 Hz, 2H), 4.06 (p, *J* = 7.0 Hz, 8H), 3.81 (s, 4H), 3.69 - 3.54 (m, 16H), 3.38 (d, *J* = 22.0 Hz, 4H), 2.86 (t, *J* = 6.0 Hz, 8H), 1.25 (t, *J* = 7.0 Hz, 12H).

**³¹P NMR:** (162 MHz, CDCl3) δ: 25.33.

**¹³C NMR:** (101 MHz, CDCl3) δ: 160.3, 151.7 (d, *J* = 8.0 Hz), 137.0, 122.4, 121.1 (d, *J* = 5.0 Hz), 70.9, 70.1, 62.3, 61.7, 54.4, 36.6 (d, *J =* 135.0 Hz), 16.5.

### Step 4:

To a stirred solution of **33** (96 mg, 0.144 mmol) in dichloromethane (10 mL) is added trimethylsilylbromide (569 µL, 4.31 mmol) dropwise at room temperature. After 18h, MeOH is added to the reaction mixture and the reaction solvent is removed immediately *in vacuo.* The residue is then re-dissolved in MeOH and precipitated with diethyl ether, to give the desired compound, MacroMePhos, as a white solid with quantitative conversion.

**HRMS (ESI-):** [M-H]- Calcd. for C26H42N4O10P2 631.2303. Found 631.2305; (Error: 0.3 ppm).

**¹H NMR:** (400 MHz, D2O) δ: 8.10 (t, *J* = 7.5 Hz, 2H), 7.66 (t, *J* = 6.5 Hz, 4H), 4.72 (s, 4H), 3.94 (br s, 8H), 3.70 (br s, 8H), 3.63 (br s, 8H), 3.46 (d, *J =* 21.0 Hz, 4H).

**³¹P NMR:** (162 MHz, D2O) δ: 17.79 (s).

**¹³C NMR:** (101 MHz, D2O) δ: 154.4, 146.3, 141.1, 126.0, 124.0, 69.5, 63.6, 55.9, 53.3, 36.6 (d, *J* = 126.0 Hz).

### II- Radiolabelling chelators of the invention with actinium

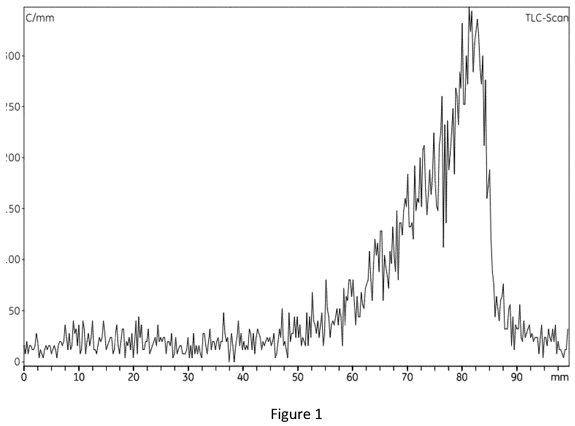

MacroPhos (0.1 mg), 10 µL gentisic acid (0.1 mg/50-100 µL tot. volume) and ²²⁵Ac³⁺ (0.09-0.3 MBq radioactivity) is added to 1 M ammonium acetate buffer (pH 6). The reaction is performed at RT and after 5 min [²²⁵Ac][Ac(MacroPhos)] is formed with 100% yield. Final concentration of the chelator is 1.7-3.3 x 10⁻³ M. TLC chromatogram is given in Figure 1.

MacroMePhos (0.1-0.2 mg), and ²²⁵Ac³⁺ (0.02 MBq radioactivity) is added to 1 M ammonium acetate buffer (pH 6). The reaction is performed at RT and after 30 min [²²⁵Ac][Ac(MacroMePhos)] is formed with 83 +/- 8% yield. Final concentration of the chelator is 1.3-2.9 x 10⁻³ M. After 24h radiochemical yield of this complex falls to 53%.

### III- Stability studies in serum

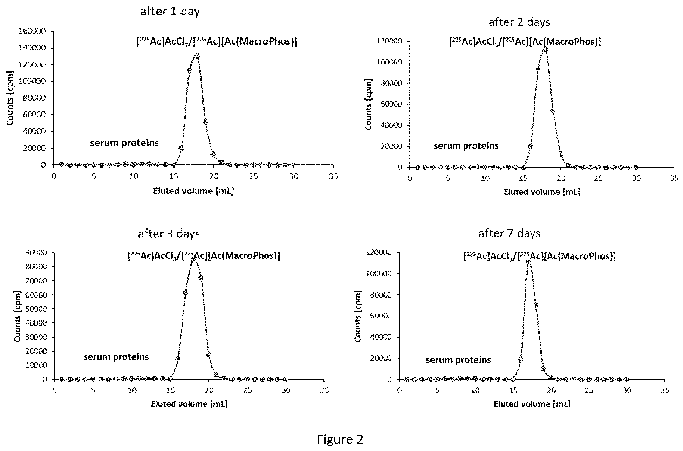

[²²⁵Ac][Ac(MacroPhos)] is mixed with human serum (1:100 vol/vol). Serum stability studies of [²²⁵Ac][Ac(MacroPhos)] are carried out up to 7 days and results are presented below (Figure 2).

Up to 7 days [²²⁵Ac][Ac(MacroPhos)] is stable (94.0 +/- 8.5% of this actinium complex remains in human serum in intact form), percentage of activity bound to serum proteins is 1.3- 1.8%.

### IV- Synthesis of functionalised Chelator of formula (III)

A biofunctionalized version of 6 is synthesized, MacroPhos-AVFA **21.**

This structure is composed of a model AVFA peptide bound to the chelator *via* metal-free click chemistry at one of the side arms.

The synthetic route to this compound is outlined below:

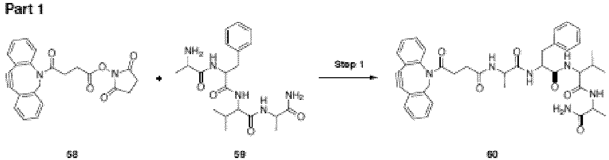

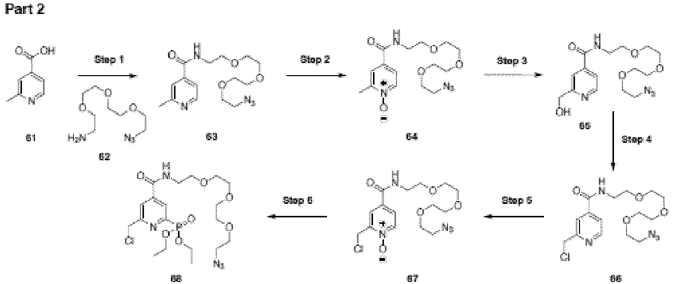

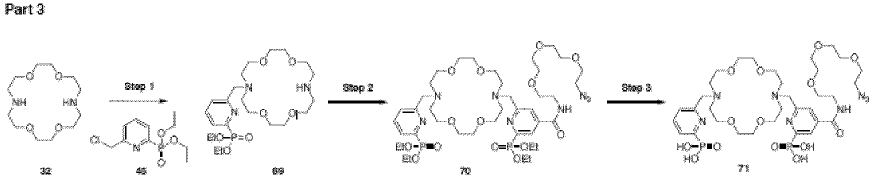

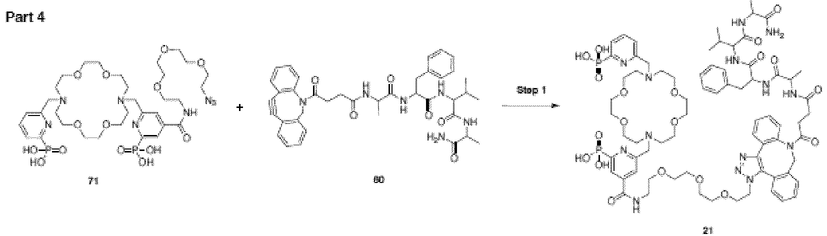

The synthesis of compound **45** is achieved following the same synthetic route outlined by Salaam *et al.* in 2018 (DOI: 10.1021/acs.inorgchem.8b00666). Compound **59** is synthesized at the University of Vienna following a procedure of Guarrochena *et al.* (https://doi.org/10.1002/psc.3488). In brief, the peptide **59** is prepared in 1 mm scale by semi-automated SPPS on a Alstra peptide synthesizer using standard Fmoc peptide chemistry on Rink amide resin. For that, the Fmoc protected amino acids (Ala-Val-Phe-Ala, 5 eq. each) are coupled one by one with Oxyma 5M in DMF (5 eq.), Fmoc deprotection steps in between are carried out using 20% piperidine in DMF. After completed coupling, the resin is Fmoc deprotected and cleaved from the resin using a cleavage cocktail of TFA, phenol, triiisopropylsilane and water. The crude product is washed with ice cold diethyl ether three times to obtain a yellowish solid in sufficient purity (93%, 200 mg, 50% yield).

### Radiolabelling with actinium

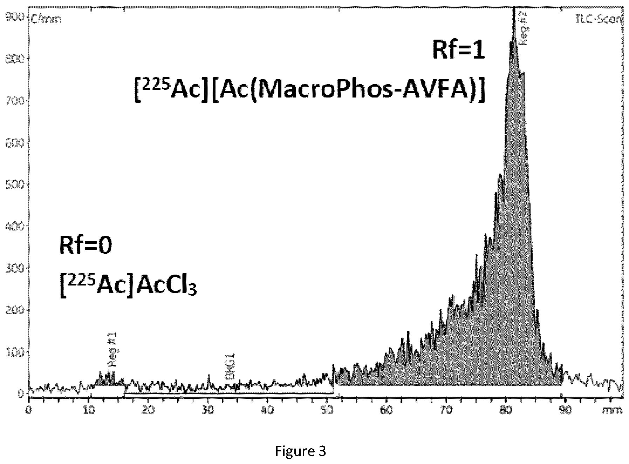

0.1 mg of bifunctionalised chelator **21,** 10 µL gentisic acid (0.1 mg/300 µL tot. volume) and ²²⁵Ac³⁺ (0.3 MBq radioactivity) is added to 1 M ammonium acetate buffer (pH 6). The reaction is performed at RT and after 5 min [²²⁵Ac][Ac(MacroPhos-AVFA)] was formed with a 99% yield. Final concentration of the chelator is 1x10⁻³ M. TLC chromatogram is given in Figure 3.

### Serum Stability Studies

[²²⁵Ac][Ac(MacroPhos-AVFA)] is mixed with human serum (1:100 vol/vol).

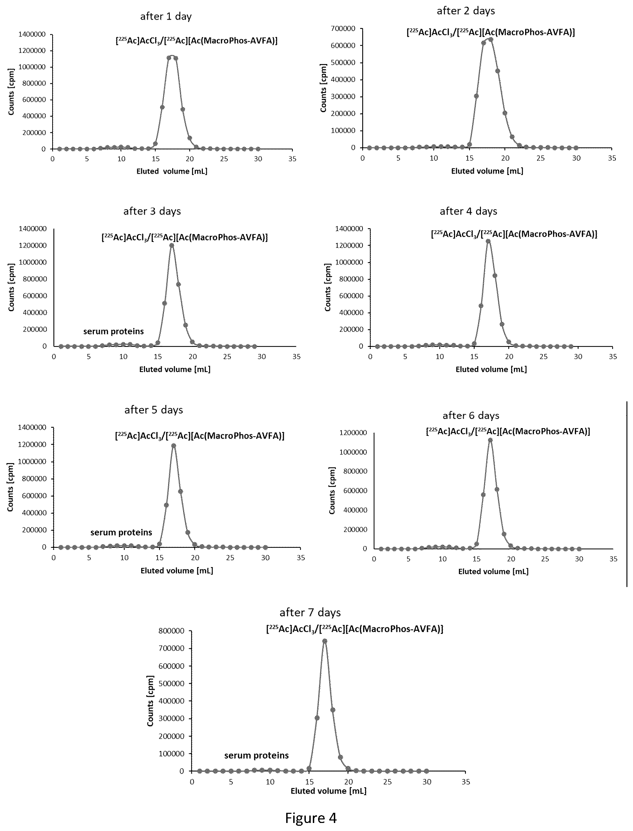

Serum stability studies of [225Ac][Ac(MacroPhos-AVFA)] are carried out up to 7 days and results are presented below (Figure 4).

Up to 7 days 92 +/- 6% of [²²⁵Ac][Ac(MacroPhos-AVFA)] are in intact form in human serum.

### Conclusions

[²²⁵Ac][Ac(MacroPhos)] demonstrates the same stability as its biofunctionalized version, up to 7 days 92 +/- 8% of [²²⁵Ac][Ac(MacroPhos)] in intact form and 92 +/- 6% of [²²⁵Ac][Ac(MacroPhos-AVFA)] in intact form. Accordingly, the attached model peptide does not influence the actinium complex stability.

## Claims

1. Chelate comprising a radioisotope and a compound of general formula (I): 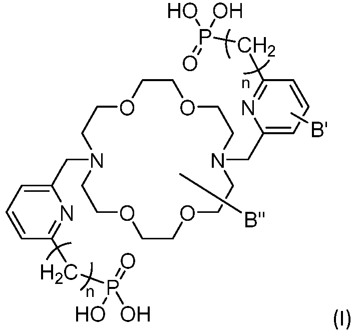
wherein n is independently chosen from 0, 1, 2, 3 or 4; preferably, n is 0 or 1; and
wherein B' is a hydrogen atom, B or -L-C, and
B" is ahydrogen atom or B,
B being preferably a saturated or unsaturated, acyclic hydrocarbon group comprising 1 to 12 carbon atoms with optionally one or more heteroatoms, and comprising a reactive group chosen from the carboxyl, acyl halide, acid anhydride, hydroxyl, aldehyde, azide, ketone, thiol, amine, amide, halogeno, ester, ether, vinyl, alkynyl (for example, propargyl), imide, maleimide and oxime groups; N-hydroxysuccinimide esters, squaric esters or isothiocyanate;
L being a linker; and
C being a biological cell-targeting moiety.

2. The chelate according to claim 1, wherein B' and B" are a hydrogen atom and wherein said compound has general formula (II):

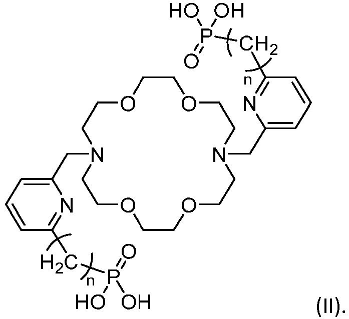

3. The chelate according to claim 1, with the proviso that when B' is B or -L-C, then B" is a hydrogen atom and when B' is a hydrogen atom, then B" is B.

4. The chelate according to claim 3, wherein said compound has a general formula (III):

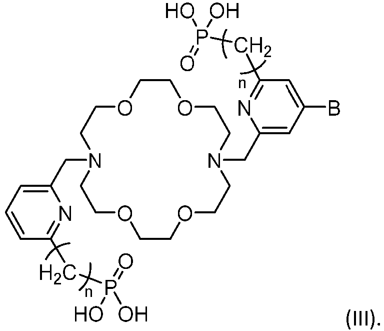

5. The chelate according to claim 3, wherein said compound has a general formula (IV):

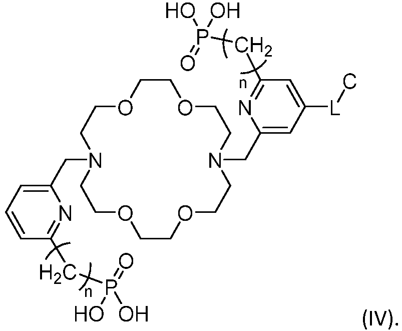

6. The chelate according to anyone of claims 1 to 5, wherein said radioisotope is selected in the group consisting of actinium such as actinium-225, terbium, such as terbium-155 or terbium-161, zirconium, such as zirconium-89, lutetium, such as lutetium-177, lanthanum, such as lanthanum-132 or lanthanum-135 and radium, such as radium-223 or radium-224.

7. The chelate according to claim 6, wherein said radioisotope is actinium-225.

8. The chelate according to anyone of claims 1 to 7 for use as a medicament.

9. The chelate according to anyone of claims 1 to 7 for use for the treatment of cancer.

10. The chelate according to anyone claims 1 or 7 for use according to claim 9 for the treatment of melanoma, glioblastoma, breast, pancreas, lung, gastro-intestinal, gynecological or prostate cancer.

11. Pharmaceutical composition comprising the chelate according to anyone of claims 1 to 7.

12. Compound of structure (IIB):

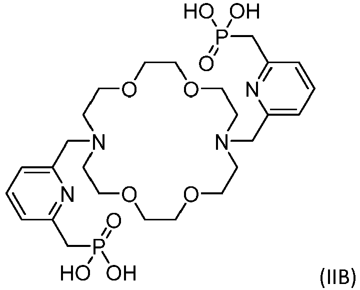

13. Use of a compound of general formula (I): 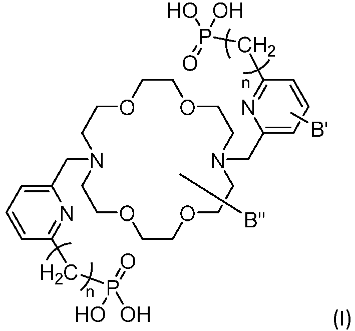
wherein n is independently chosen from 0, 1, 2, 3 or 4; preferably, n is 0 or 1;
wherein B' is a hydrogen atom, B or -L-C, and
B" is a hydrogen atom or B,
B being preferably a saturated or unsaturated, acyclic hydrocarbon group comprising 1 to 12 carbon atoms with optionally one or more heteroatoms, and comprising a reactive group chosen from the carboxyl, acyl halide, acid anhydride, hydroxyl, aldehyde, azide, ketone, thiol, amine, amide, halogeno, ester, ether, vinyl, alkynyl (for example, propargyl), imide, maleimide and oxime groups; N-hydroxysuccinimide esters, squaric esters or isothiocyanate;
L being a linker; and
C being a biological cell-targeting moiety,
to chelate a radioisotope.

14. The use a of a compound of general formula (I) according to claim 13, wherein said radioisotope is selected in the group consisting of actinium such as actinium-225, terbium, such as terbium-155 or terbium-161, zirconium, such as zirconium-89, lutetium, such as lutetium-177, lanthanum, such as lanthanum-132 or lanthanum-135 and radium, such as radium-223 or radium-224.

15. The use of a compound of general formula (I) according to claim 14, wherein said radioisotope is actinium-225.
